# EUROPEAN PATENT SPECIFICATION

(11) **EP 1 207 934 B1**
(45) Date of publication and mention of the grant of the patent: **06.08.2014**
(21) Application number: 00961620.2
(22) Date of filing: 01.09.2000
(51) Int. Cl.: A61M 37/00

(54) **GUIDABLE INTRAVASCULAR BLOOD PUMP**
LENKBARE INTRAVASKULARE BLUTPUMPE
POMPE A SANG INTRAVASCULAIRE POUVANT ETRE GUIDEE

(30) Priority: 03.09.1999 US 152249 P
(43) Date of publication of application: 29.05.2002
(73) Proprietor: A-Med Systems, Inc., West Sacramento, CA 95691-3817 (US)
(72) Inventor: ABOUL-HOSN, Walid, N., Fair Oaks, CA 94628 (US); KANZ, William, R., Sacramento, CA 95822 (US); BAKER, Bruce, Placerville, CA 95667 (US)
(74) Representative: Eckert, Lars
(86) International application number: PCT/US2000/024515
(87) International publication number: WO 2001/017581

(56) References cited:
- EP-A- 0 916 359
- WO-A-89/05164
- CA-A1- 2 256 427
- DE-A1- 19 622 335
- US-A- 3 585 983
- US-A- 4 895 557
- US-A- 5 163 910
- US-A- 5 692 882
- US-A- 5 746 709
- US-A- 5 746 709

## Description

### Field of the Invention

The present invention relates generally to blood pumps and, more particularly, to an improved intra-vascular blood pump having a guide mechanism which provides the ability to selectively guide the intravascular pump to a desired location within a patient's circulatory system.

### Description of Related Art

Over the years, various types of blood pumps have been developed for the purpose of augmenting or replacing the blood pumping action of damaged or diseased hearts. Blood pumps are commonly used in three situations: (1) for acute support during cardio-pulmonary operations; (2) for short-term support while awaiting recovery of the heart from surgery; or (3) as a bridge to keep a patient alive while awaiting heart transplantation. The pumps may be designed to provide right and/or left ventricular assist, although left ventricle assist is the most common application in that it is far more common for the left ventricle to become diseased or damaged than it is for the right ventricle.

Blood pumps must provide leak-free operation and must avoid contamination of the fluid by the pump components and the external environment. Such pumps must also pump the fluid at a suitable rate without applying excessive Reynolds shear stress to the fluid. It is well known to those skilled in the art that lysis or cell destruction may result from application of shear stress to cell membranes. Red blood cells are particularly susceptible to shear stress damage as their cell membranes do not include a reinforcing cytoskeleton to maintain cell shape. Lysis of white blood cells and platelets also occurs upon application of high shear stress. Lysis of red blood cells can result in release of cell contents which trigger subsequent platelet aggregation. Sublytic shear stress leads to cellular alterations and direct activation and aggregation of platelets and white blood cells.

Intravascular blood pumps comprise miniaturized blood pumps capable of being percutaneously or surgically introduced into the vascular system of a patient, typically to provide left and/or right heart support. One type of intravascular pump is an axial flow blood pump comprising a cable-mounted rotor surrounded by a protective shroud. The pump, along with the rotor and shroud, are mounted at the end of an elongated flexible catheter. The catheter is inserted into the aorta from a remote entry point, such as an incision below the groin that provides access into a femoral artery. The catheter then passes through the descending aorta until it reaches the ascending aorta, near the heart. The catheter device encloses a rotating drive cable which is coupled to the impeller blade at one end, and which emerges from the exposed end of the catheter, near the patient's groin, at the other end. When the exposed end of the drive cable is mechanically rotated, using a device located outside the patient's body, it conveys the rotational force through the length of the catheter, causing the impeller to spin at high speed near the heart. This type of blood pump finds particular application in providing ventricular assist during surgery or providing temporary bridging support to help a patient survive a crisis.

While generally effective in providing ventricular assisting functions, prior art intravascular blood pumps nonetheless suffer various drawbacks. A significant drawback is that prior art intravascular blood pumps are difficult to guide into the appropriate position within the circulatory system of a patient. This is due largely to the fact that the elongated catheter is incapable of providing the degree of control necessary to easily negotiate the pump through the tortuous pathways leading up to and into the heart. When attempting to place the blood pump in a trans-valvular configuration (with the inlet in the left ventricle and the pump outlet in the ascending aorta), the natural tendency of the catheter to stay straight may cause the pump to be inadvertently placed in the carotid ostia, which can be dangerous if the pump is operated to withdraw blood from the brain.

To overcome these difficulties, certain guide mechanisms may be employed to assist the physician placing the pump in the appropriate position within the circulatory system. One type of supplemental guide mechanism is a guide catheter. Guide catheters are designed with certain guidability characteristics such that physicians can selectively position them within the vasculature or heart with relative ease. A central lumen is provided within the guide catheter such that the intravascular pump may be introduced therein and guided while it is advanced towards the predetermined circulatory site. While generally effective at providing a guiding feature for such intravascular blood pumps, employing such supplemental guide mechanisms is nonetheless disadvantageous in that they consume valuable space within the vessels. A guide catheter, for example, would necessarily be larger in diameter than the diameter of the pump and protective shroud in order to provide adequate passage of those components. As will be appreciated, this restricts the amount of space available for blood to flow within the particular vessel, and increases the size of the required puncture wound for accessing the vessel.

DE 196 22 335 discloses a balloon catheter comprising a catheter hose, a balloon support connected to the catheter hose and an expandable balloon carried on the balloon support. A small diameter pump is disposed between the catheter hose and the balloon support for pumping blood.

WO 89/05164 discloses an inflow cannula for retrograde insertion of the blood intake for an intravascular blood pump through the aortic valve. The cannula comprises a stiff body section adapted to be attached at its trailing end to the intake of the blood pump and a substantially soft, flexible, resilient tip section at the leading end of the body section.

EP-A-0,916,359 discloses a cannula device for use on the inflow end of an implantable heart pump.

The present invention is directed at eliminating and/or reducing the effects of the foregoing drawbacks of prior art intravascular blood pumps.

### Summary of the Invention

The present invention provides an intravascular blood pump system for ventricular assist according to claim 1.

The present invention overcomes the drawbacks of the prior art by providing an improved intravascular blood pump equipped with integrated features for selectively guiding the intravascular blood pump to a predetermined location in the patient's circulatory system, i.e. heart and/or vasculature. In so doing, the intravascular blood pump of the present invention eliminates the need for supplemental guiding mechanisms, such as a separate, large diameter guide catheter as used in the prior art.

In a first broad aspect of the present invention, an intravascular blood pump system is provided comprising an intravascular blood pump having a cannula coupled thereto and an "over-the-wire" type guide mechanism for selectively positioning the intravascular blood pump and cannula at a predetermined location within the circulatory system of a patient. To accomplish this, a central lumen is formed through at least a portion of the intravascular blood pump system such that a guide wire may be progressed therethrough and advanced to the predetermined location in the circulatory system of the patient. After the guide wire is advanced to this desired location, the intravascular blood pump and cannula may thereafter be advanced along the guide wire to the desired location.

In a second broad aspect of the present invention, an intravascular blood pump system is provided comprising an intravascular blood pump having a cannula coupled thereto and a "side-rigger" or "rapid exchange" type guide mechanism for selectively positioning the intravascular blood pump and cannula at a predetermined location within the circulatory system of a patient. To accomplish this, a side lumen is formed along a length of at least one of the intravascular blood pump and the cannula. A guide guide wire may be advanced to the predetermined location in the circulatory system of the patient. After the guide wire is advanced to this desired location, the intravascular blood pump and cannula may thereafter be advanced along the guide wire to the desired location.

In a further embodiment, the intravascular blood pump includes a rotor, a shroud for receiving the rotor, and a drive cable coupled to the rotor for driving the rotor within the shroud.

In a further embodiment, the cannula is coupled to the shroud of the intravascular blood pump.

In a further embodiment, the drive cable sheath is provided having a central lumen for receiving the drive cable, and wherein a purge fluid delivery system is coupled to the drive cable sheath to deliver purge fluid to the rotor.

In a further embodiment, the drive cable sheath includes at least one side lumen for delivering the purge fluid towards the rotor.

In a further embodiment, a portion of the purge fluid is delivered through the at least one side lumen and past the rotor, and a portion of purge fluid is rerouted back from the rotor through the central lumen of the drive cable.

A blood pressure detection mechanism is provided to detect the pressure of the blood proximate at least one of the intravascular blood pump and cannula.

The blood pressure detection mechanism comprises a fluid filled column, e.g., disposed within at least a portion of the cannula.

In a further embodiment, the guide wire is disposed at least partially within the cannula.

In a further embodiment, the guide wire is for passage through a side lumen formed in the cannula.

In a further embodiment, the guide element comprises a selectively deformable element disposed at least partially within the cannula.

The intravascular blood pump and cannula may be selectively advanced to the predetermined location within the vasculature of the patient by first passing the guide wire to the predetermined location and thereafter sliding the intravascular blood pump and cannula along the guide wire to the predetermined location.

In a further embodiment, the guide wire is for passage through a lumen extending through the drive cable and rotor.

The intravascular blood pump and cannula may be selectively advanced to the predetermined location within the vasculature of the patient by first passing the guide wire to the predetermined location and thereafter sliding the intravascular blood pump and cannula along the guide wire to the predetermine location.

In a further embodiment, the guide element comprises a guide wire and a balloon catheter.

### Brief Description of the Drawings

Many advantages of the present invention will be apparent to those skilled in the art with a reading of this specification in conjunction with the attached drawings, wherein like reference numerals are applied to like elements and wherein:
FIG. 1 is a partial sectional view of a human heart illustrating an intravascular blood pump system having an "over-the-wire" type guide mechanism according to a first broad aspect of the present invention positioned, by way of example, in a trans-valvular configuration to provide left-heart assist;
FIG. 2 is side view of the guidable intravascular blood pump system of the type shown in FIG. 1 including a motor coupler and purge fluid delivery system according to an exemplary embodiment of the present invention;
FIG. 3 is a cross-sectional view illustrating an exemplary construction of the blood pump, drive cable assembly, and cannula of the intravascular blood pump system according to the first broad aspect of the present invention;
FIG. 4 is a cross-sectional view taken along lines 4-4 of FIG. 3 illustrating an exemplary construction of the drive cable assembly and guide mechanism according to the first broad aspect of the present invention;
FIG. 5 is a cross-sectional view illustrating an exemplary construction of the motor coupler and purge fluid delivery system according to the first broad aspect of the present invention;
FIG. 6 is a partial sectional view of a human heart illustrating an intravascular blood pump system having a "rapid exchange" or "side-rigger" type guide mechanism according to a second broad aspect of the present invention positioned, by way of example, in a trans-valvular configuration to provide left-heart assist;
FIG. 7 is side view of the guidable intravascular blood pump system of the type shown in FIG. 6 including a motor coupler and purge fluid delivery system according to an exemplary embodiment of the present invention;
FIG. 8 is a cross-sectional view taken along lines 8-8 of FIG. 7 illustrating the "side-rigger" or "rapid exchange" type guide mechanism according to the second broad aspect of the present invention;
FIG. 9 is a cross-sectional view of the type shown in FIG. 8 illustrating an alternate configuration of the guide mechanism according to the second broad aspect of the present invention, and
FIG. 10 is a partial sectional view of a human heart illustrating an alternate intravascular blood pump system having an "over-the-wire" type guide mechanism according to the first broad aspect of the present invention positioned, by way of example, in a trans-valvular configuration to provide right-heart assist.

### Detailed Description of the Invention

Illustrative embodiments of the invention are described below. In the interest of clarity, not all features of an actual implementation may be described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure.

The present invention involves an intravascular pump system for use in a number of broad ranging applications involving the augmentation of blood flow within the circulatory system of a patient. As will be described below, the intravascular blood pump system of the present invention overcomes the drawbacks of the prior art by providing a guide mechanism as part of the intravascular blood pump. This advantageously allows the intravascular blood pump to be selectively guided to a predetermined location within the circulatory system of a patient without the need for bulky supplemental guide mechanisms, such as a separate guide catheter.

The intravascular pump assembly of the present invention is particularly suited for trans-valvular use, such as for left and/or right ventricular assist procedures. By way of example only, such ventricular assist procedures may be employed in cardiac operations including, but not limited to, coronary bypass graft (CABG), cardio-pulmonary bypass (CPB), open chest and closed chest (minimally invasive) surgery, bridge-to-transplant and/or failure-to-wean-from-bypass situations. It is to be readily understood, however, that the intravascular blood pump assembly and methods of the present invention are not to be limited to such applications. Moreover, while illustrated and described largely with reference to left-heart assist applications, it is to be readily understood that the principles of the present invention apply equally with regard to right-heart assist application, which are contemplated as within the scope of the present invention. These and other variations and additional features will be described throughout.

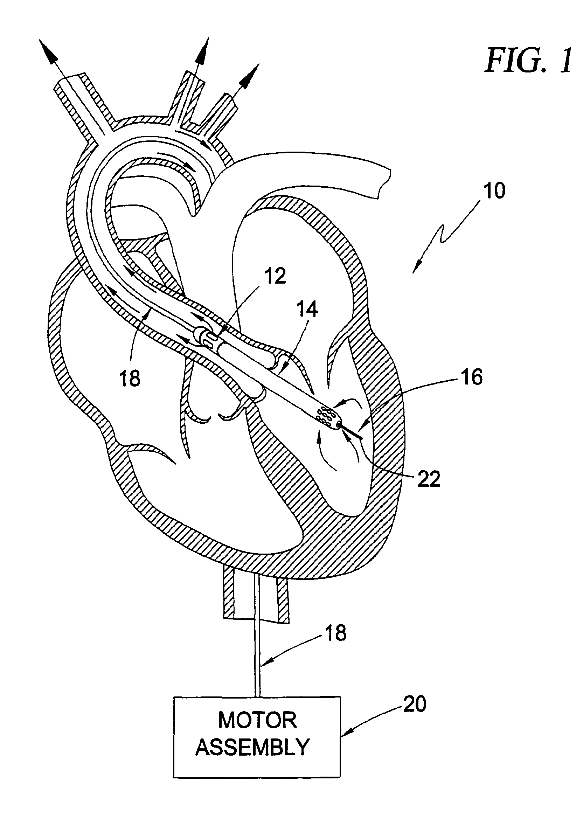

Referring to FIG. 1, shown is a guidable intra-vascular blood pump system 10 according to a first broad aspect of the present invention shown, by way of example only, in a left-heart assist configuration within a human heart. The system 10 includes an intravascular blood pump 12, a cannula 14, and an "over-the-wire" type guide mechanism 16. A drive cable assembly 18 and a motor assembly 20 are provided to drive the intravascular blood pump 12. The "over-the-wire" guide mechanism 16 comprises a suitable guide element dimensioned to pass slideably through a central lumen extending through the drive cable 18, blood pump 12, and cannula 14. The guide element is shown as a guide wire 22. According to the present invention, the "over-the-wire" guide mechanism 16 provides the ability to selectively guide the blood pump 12 and cannula 14 to a predetermined position in the circulatory system of a patient, such as the trans-valvular position shown.

To accomplish this, the guide wire 22 is first introduced into the vascular system of a patient through any suitable access point, such as through the use of the well known Seldinger technique. The guide wire 22 can then be advanced within the patient to a desired location within the circulatory system of the patient. This may be done using the control features of the guide wire 22 itself, or may be facilitated through the use of any number of supplemental guidance mechanisms or techniques to ensure the proper and efficient placement of the guide wire 22. Such supplemental guidance techniques may include, but are not necessarily limited to, guide catheters and/or techniques involving ultra-sound or fluoroscopy. Once the guide wire 22 is positioned at the desired location (such as in left ventricle as shown), the blood pump 12 and cannula 14 may thereafter be advanced along the guide wire 22 and positioned in the trans-valvular configuration shown. Under the operation of the motor assembly 20, the blood pump 12 may be used for left-heart assist by selectively withdrawing blood from the left ventricle (through the interior of the cannula 14) for delivery outward through outflow apertures formed in the blood pump 12. This outflow from the blood pump 12 flows along the exterior of the drive cable assembly 18 in a substantially axial fashion for arterial distribution throughout the body.

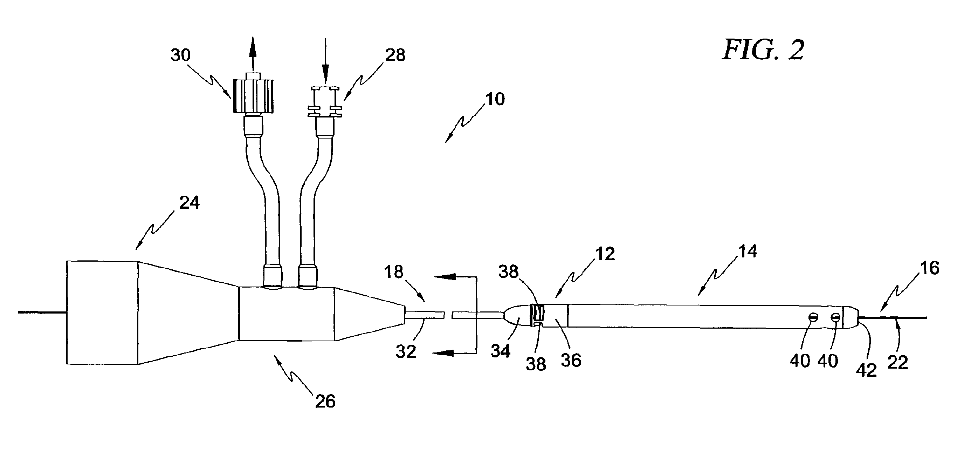

Referring to FIGS. 2-5, an exemplary embodiment of the intravascular blood pump system 10 of FIG. 1 will now be described. As shown in FIG. 2, the intravascular blood pump system 10 includes a motor coupler 24 and, as will be described in greater detail below, a purge fluid delivery system 26 for providing a two-way fluid flow within the drive cable assembly 18 during pump operation. The purge fluid delivery system 26 includes a fluid inlet conduit 28 for introducing pressurized purge fluid from a fluid source (not shown) for delivery into the blood pump 12, and a fluid outlet conduit 30 to withdraw a return flow of purge fluid from the blood pump 12. The motor coupler 24 establishes a mechanical connection between a motor (not shown) and a drive cable (not shown) for providing motive force to the blood pump 12 for pump operation. The drive cable assembly 18 includes a drive cable sheath 32 which, in addition to serving a purge fluid delivery function, also serves as a protective housing for the drive cable (not shown). Although shown in broken form for clarity, it will be appreciated that the drive cable assembly 18 (and all components thereof) may be provided in any suitable length sufficient for intravascular applications. That is to say, the length of the drive cable assembly 18 must be enough to reach between the motor coupler 24 and purge fluid delivery system 26, located outside the patient, and the desired location within the patient's circulatory system where the blood pump 12 is to be positioned.

The intravascular blood pump 12 is shown (by way of example only) as an axial flow intravascular blood pump. The blood pump 12 includes pump body 34, a rotor shroud 36 having flow ports 38, and an internally disposed rotor (not shown) having a shaft rotatably disposed within the pump body 34 and an impeller rotatably disposed within the rotor shroud 36. The cannula 14 is fixedly attached to the rotor shroud 36 and may extend any suitable length therefrom depending upon the particular intravascular application. The cannula 14 preferably includes a plurality of ports or fenestrations 40 about its distal region, as well as an end port 42, which allow for the ingress or egress of blood into or from the cannula 14 depending upon the operation of the blood pump 12. That is to say, if the pump 12 is configured for left-heart assist as shown in FIG. 1, then the ports 40, 42 will allow the ingress of blood into the cannula 14 from the left ventricle. If, on the other hand, the blood pump 12 is configured for right-heart assist (i.e. with the pump 12 in the right atrium and the distal end of the cannula 14 located within the pulmonary artery), then the ports 40, 42 will allow the egress of blood from the cannula 14 into the pulmonary artery. (Details on right-heart assist applications will be discussed in greater detail below.) The pump 12 and cannula 14 may be dimensioned to any suitable diameter for intravascular applications. For example, the range of sizes may include, but is not necessarily limited to, 3 to 10 mm (9 French to 30 French), although the range is more preferably from 4.7 to 8 mm (14 French to 24 French), and most preferably from 6 to 6.7 mm (18 French to 20 French).

The "over-the-wire" type guide mechanism 16 includes the guide wire 22 and, as will be explained in greater detail below, a central lumen extending through the cannula 14, blood pump 12, drive cable assembly 18, purge fluid delivery system 26, and motor coupler 24. As noted above, the central lumen is dimensioned to slideably receive the guide wire 22 such that the blood pump 12 and cannula 14 may be slideably advanced along the guide wire 22 to a desired location within the circulatory system of a patient after the guide wire 22 has been so positioned using conventional guidance techniques. It is to be readily understood that, while shown as a conventional guide wire 22, imaging wires may be used.

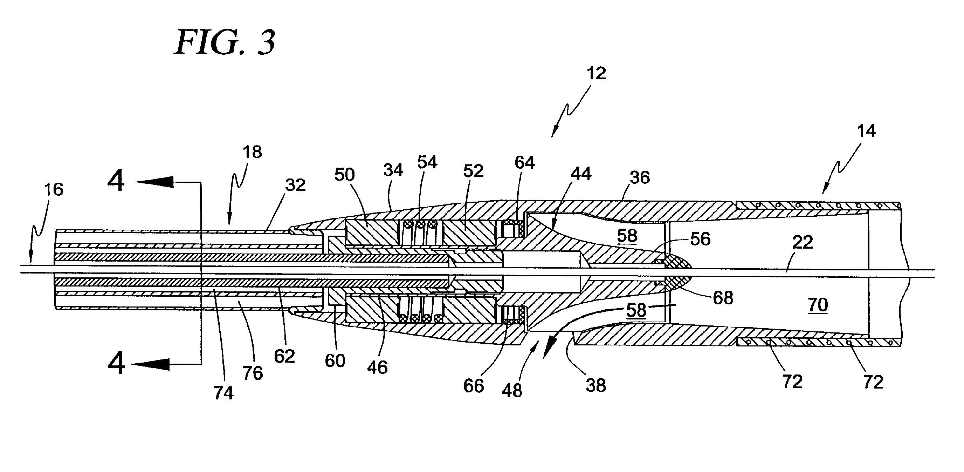

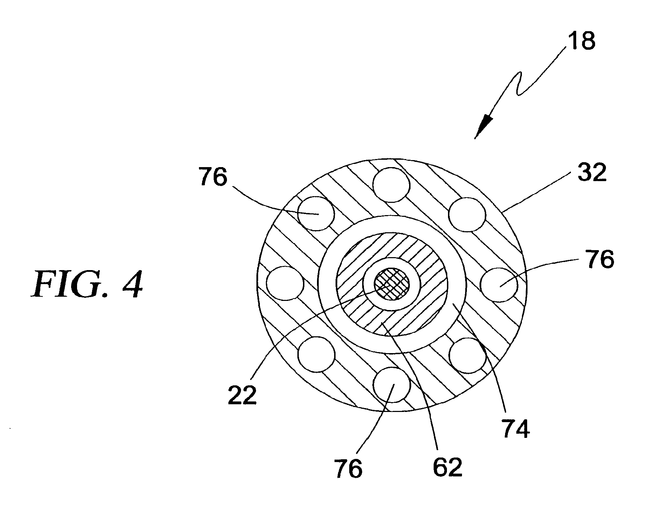

FIGS. 3 and 4 further detail the construction of the blood pump 12, cannula 14, drive cable assembly 18, and "over-the-wire" guide mechanism 16. The blood pump 12 includes a rotor 44 having a shaft 46 and an impeller 48. The shaft 46 is rotatably disposed within the pump body 34 via a bearing pack comprising, by way of example, ball bearing assemblies 50, 52 and spring 54. Ball bearings assemblies 50, 52 are well known in the art, each comprising an inner race which rotates along with the rotor shaft 46, an outer race which remains in a static and fixed position against the inner surface of the pump body 34, and a plurality of ball bearings disposed between the inner and outer races. The spring 54 biases each bearing assembly 50, 52 axially away from one another to reduce axial play during pump operation. The shaft 46 is generally hollow and dimensioned to receive a cable adapter 60 therein for the purpose of coupling the rotor 44 to a drive cable 62 forming part of the drive cable assembly 18. The drive cable 62 may be secured to the cable adapter 60 in any number of suitable fashions, including but not limited to the use of adhesives, crimping, and laser welding. These same techniques may be used to secure the cable adapter 60 within the shaft 46 of the rotor 44. A radial seal 64 is provided in between the wall of the pump body 34 and a distal stepped region 66 on the rotor shaft 46, the function of which will be described below.

The impeller 48 includes a hub 56 and a plurality of blades 58 extending therefrom. The hub 56 is generally conical and, according to the first broad aspect of the present invention, is hollow throughout to form part of the central lumen of the guide mechanism 16. In this regard, the hub 56 is preferably provided with a gasket or seal member 68 at its distal tip. The seal member 68 may be made of any suitable sealing material (including but not limited to silicone) such that the pump 12 and cannula 14 may be easily progressed along the guide wire 22 for delivery to a desired circulatory site. The seal member 68 should also be robust enough to prevent the ingress of blood into the interior of the rotor hub 56 during pump operation, whether the guide wire 22 remains in place or is fully withdrawn. The blades 58 are dimensioned to reside in close tolerance with the interior surface of the shroud 36. In operation, the blades 58 impart both an axial and radial vector on the blood which causes it to flow outward through the flow ports 38 formed in the shroud 36. As used herein, the term "axial flow" is deemed to include flow characteristics like that shown in FIG. 3, which include both an axial and slight radial component. It is to be readily appreciated that, although shown as an axial flow type, blood pump 12 may comprise any number of suitable types of intravascular blood pumps, including but not limited to so-called "mixed flow" intravascular blood pumps without departing from the scope of the present invention.

The cannula 14 is coupled at its proximal end to the rotor shroud 36. This may be accomplished in any number of fashions, including but not limited to the use of adhesives. This may also be facilitated by dimensioning the shroud 36 to include a narrow inlet region 70 capable of being received flushly within the proximal end of the cannula 14. The inlet region 70 of the shroud 36 should preferably have a tapered interior surface for establishing a smooth flow transition between the cannula 14 and the region containing the impeller blades 58. Although shown as a single integral element, it is to be understood that the pump body 34 and shroud 36 may comprise two separate (and sometimes separable) components, the significance of which will become apparent below. The pump body 34 and shroud 36 may be constructed from any number of suitable materials, including but not limited to stainless steel or other medical grade compositions or alloys. The cannula 14 may also be constructed from any number of suitable materials, including but not limited to medical grade plastics. As shown, the cannula 14 may also be fortified with spiral-wound reinforcement wire 72 within the walls of the cannula 14.

The drive cable assembly 18 includes the drive cable 62 and the drive cable sheath 32. The drive cable 62 is coupled to the rotor 44 via the cable adapter 60. The drive cable sheath 32 includes a central lumen 74 and a plurality of side lumens 76. The central lumen 74 serves as a protective covering for the drive cable 62. The central lumen 74, along with the side lumens 76, also forms part of the purge fluid delivery system 26 shown above in FIG. 2, which will be described in greater detail below. The side lumens 76 are provided in fluid communication with the fluid inlet conduit 28, while the central lumen 74 is provided in fluid communication with the fluid outlet conduit 30. The side lumens 76 are thus configured to deliver purge fluid into the pump 12, while the central lumen 74 is configured to transport purge fluid away from the pump 12 along the length of the drive cable 62.

The pressurized purge fluid within the side lumens 76 may take one of two flow paths upon entry into the pump 12. One flow path passes through the interior of the pump 12 and onward past the radial seal 64 to prevent the ingress of blood into the pump body 34 during pump operation. More specifically, the purge fluid flows distally around the cable adapter 60, through the ball bearing assemblies 50, 52, and onward past the radial seal 64. This egress of purge fluid past the radial seal 64 can be controlled to effectively thwart the ingress of blood past the radial seal 64, which might otherwise cause clotting and/or pump damage. The other flow path is directed back out the central lumen 74 for delivery to the fluid outlet conduit 30. In so doing, this flow path bathes the components of the pump 12 and/or drive cable 62 and thereby reduces frictional heating within the pump 12 and/or the central lumen 74 of the sheath 32 during pump operation.

The "over-the-wire" guide mechanism 16 includes a central lumen through which the guide wire 22 may extend for the purpose of slideably advancing the blood pump 12 and cannula 14 into a desired position within the circulatory system of a patient. In the embodiment shown, this central lumen is established by forming and co-aligning the individual central lumens within each of the drive cable 62, the cable adapter 60, the shaft 46 and hub 56 of the rotor 44, and the cannula 14. In this regard, the drive cable 62 is preferably of wound-wire construction having a central lumen formed therein. The central lumens within the cable adapter 60, rotor 44, and gasket 68 may be formed via machining or molding processes. These central lumens should preferably be sized such that they permit the slideable passage of the pump 12 and cannula 14 therealong, but do not interfere with or constrain the guide wire 22 to cause inadvertent rotation of the guide wire 22 during pump operation. As noted above, it is also contemplated to remove the guide wire 22 after the pump 12 and cannula 14 are properly positioned in the patient. In this case, the gasket or seal 68 on the hub 56 should be robust enough to reseal after the guide wire 22 is withdrawn and prevent the ingress of blood into the interior of the rotor 44.

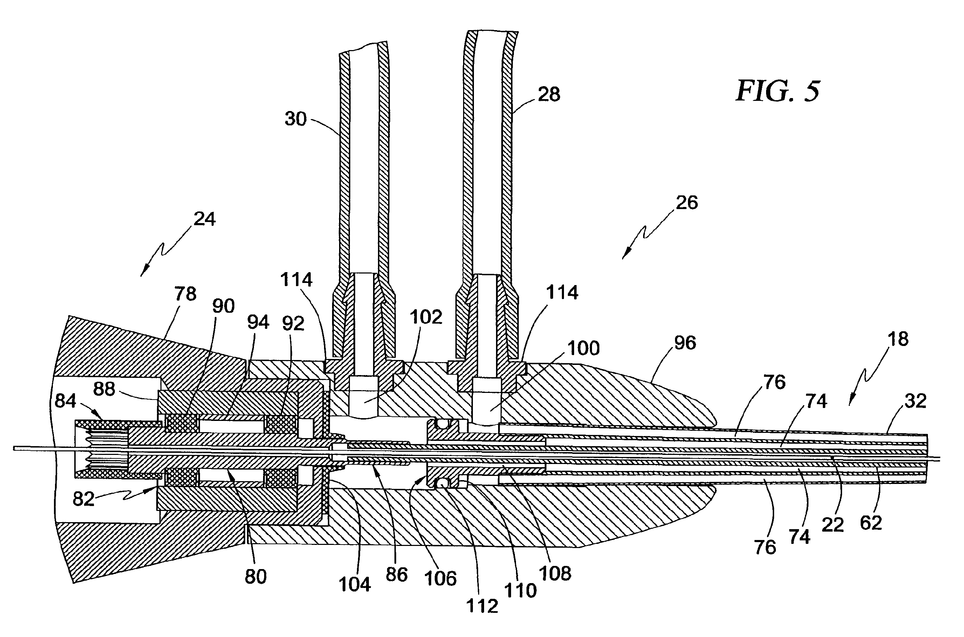

Referring to FIG. 5, the motor coupler 24 includes a housing 78, a drive shaft adapter 80, and a bearing assembly 82. The drive shaft adapter 80 includes a drive shaft coupler 84 dimensioned to receive a drive shaft of a motor (not shown), and a drive cable coupler 86 dimensioned to receive the drive cable 62. Any of a variety of attachment techniques may be employed to securely fasten the drive cable 62 to the drive cable coupler 86, including but not limited to adhesives, crimping, and laser welding. The drive shaft adapter 80 is rotatably disposed within the housing 78 by the bearing assembly 82. The bearing assembly 82 includes a sleeve 88 (which may alternatively be formed as an integral part of the housing 78) for retaining a pair of ball bearing assemblies 90, 92 and a spring 94 of the type described above. That is, each bearing assembly 90, 92 generally comprises an inner race which rotates along with the drive shaft adapter 80, an outer race which remains in a static and fixed position against the inner surface of the retaining sleeve 88, and a plurality of ball bearings disposed between the inner and outer races. The spring 94 is provided to bias each bearing assembly 90, 92 axially away from one another to reduce axial play during operation.

The purge fluid delivery system 26 includes a housing 96 having a central lumen 98, an inflow port 100, and an outflow port 102. The housing 96 is also dimensioned to matingly receive a portion of the motor coupler 24. In this regard, a seal element 104 is provided sandwiched in between the housing 96 and housing 78 and including an aperture which extends about the drive shaft adapter 80 as it exits the housing 78 to prevent the ingress of purge fluid into the motor coupler 24. A fluid guide structure 106 is also provided within the central lumen 98 for the purpose of separating the inflow and outflow ports 100, 102. The fluid guide structure 106 includes a central lumen 108 through which the drive cable 62 extends, and an elevated portion 110 that retains an O-ring 112 against the inner surface of the central lumen 98 of the housing 96. The drive cable sheath 32 is secured to the housing 96 such that the inflow port 100 is communicatively coupled to the side lumens 76, and the outflow port 102 is communicatively coupled to the central lumen 74. In this fashion, pressurized purge fluid may be introduced through the inflow port 100 via inflow conduit 28, and removed through the outflow port 102 via outflow conduit 30. By way of example, the inflow conduit 28 and outflow conduit 30 may be coupled to their respective ports 100, 102 via barbed connectors 114. Similarly, the inflow and outflow conduits 28, 30 may be equipped with any number of suitable connectors (such as those illustrated by way of example in FIG. 2) for establishing fluid communication with a source of pressurized fluid (not shown). The pressurized fluid source (not shown) may include, but is not necessarily limited to, the use of a syringe, an indeflator, a fluid delivery pump, or an accumulator arrangement to provide the requisite delivery of pressurized fluid. The purge fluid delivery system 26 thus provides a two-way transmission of purge fluid within the drive cable sheath 32 for the purposes of cooling the blood pump 12 and preventing the ingress of blood past the radial seal 64 and into blood pump 12.

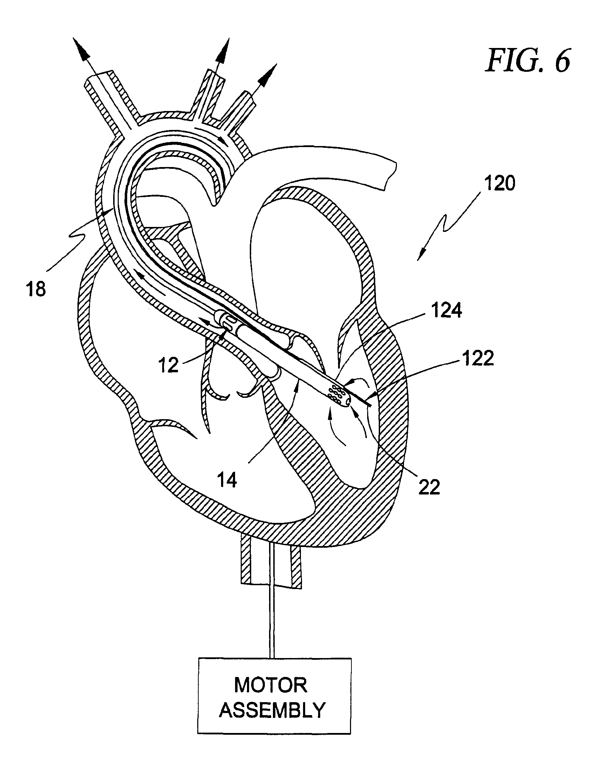

Referring to FIG. 6, shown is a guidable intra-vascular blood pump system 120 according to a second broad aspect of the present invention. As will be described hereinafter, the intravascular blood pump system 120 differs from the intravascular blood pump system 10 described above only as to the type of guide mechanism employed. In the interest of clarity and consistency, then, like reference numerals will be used to denote like elements and distinctions pointed out where necessary. Moreover, due to the commonality of principles employed in both intravascular blood pump systems 10, 120, a discussion to the level of detail set forth above is not deemed necessary with regard to the intravascular blood pump system 120. Instead, those aspects in common with the intravascular blood pump 10 are hereby incorporated into the discussion of the intravascular blood pump system 120.

In its most general form, the intravascular blood pump system 120 of this second broad aspect of the present invention comprises the blood pump 12 and cannula 14 arrangement, wherein the cannula 14 is equipped with a "side-rigger" or "rapid exchange" guide mechanism 122. In an important aspect of the present invention, the "rapid exchange" or "side-rigger" guide mechanism 122 includes a guide carriage 124 formed along at least a portion of the cannula 14, and a guide wire 22 dimensioned to pass slidably through a lumen (not shown) extending through the guide carriage 124. The "rapid exchange" guide mechanism 122 thereby provides the ability to selectively guide the blood pump 12 and cannula 14 to a predetermined position in the circulatory system of a patient in the manner described above. Namely, the guide wire 22 may be first introduced into the vascular system of a patient through any suitable access point and guided to a desired location within the circulatory system of the patient, i.e. the left ventricle as shown. The blood pump 12 and cannula 14 may thereafter be advanced along the guide wire 22 and positioned in the trans-valvular configuration shown for providing left-heart assist.

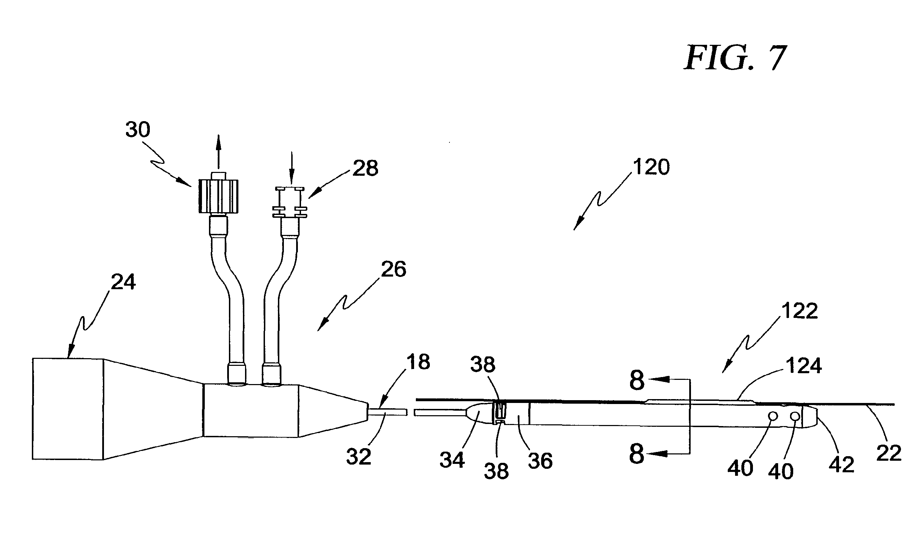

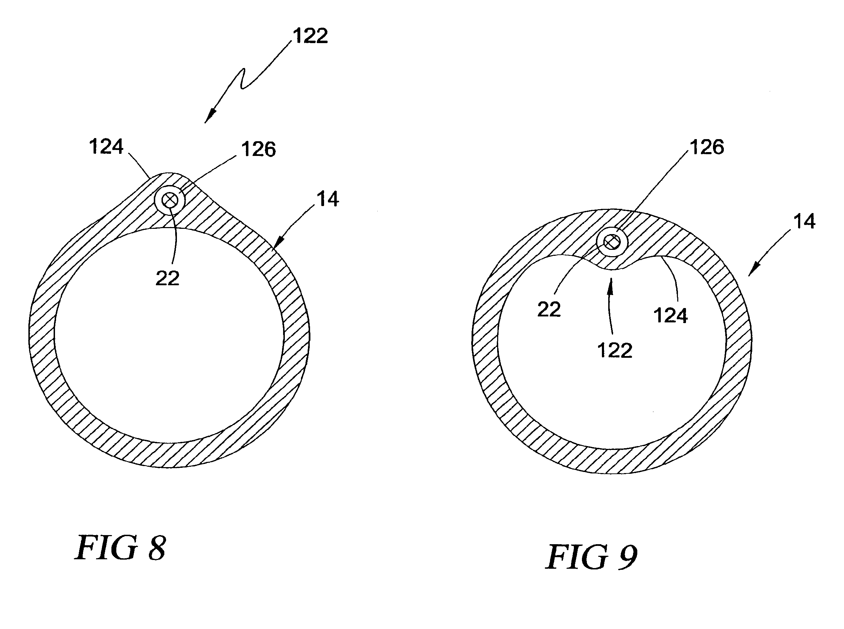

FIGS. 7-9 further illustrate the "side-rigger" or "rapid-exchange" guide mechanism 122 of this second broad aspect of the present invention. In a preferred embodiment, the "side-rigger" guide mechanism 122 includes a lumen 126 formed within the guide carriage 124. The guide carriage 124 is preferably formed as an integral extension of the wall of the cannula 14. FIGS. 7 and 8 comport with the embodiment shown in FIG. 6, namely illustrating the guide carriage 124 formed along the exterior surface of the cannula 14. FIG. 9 illustrates an alternate embodiment wherein the guide carriage 124 may be formed along the interior surface of the cannula 14. In either case, the guide wire 22 is advanced to a desired location in the vasculature of the patient, after which point the blood pump 12 and cannula 14 can be slidably advanced therealong for delivery to the desired location according to the present invention. The guide wire 22 may thereafter be withdrawn from the patient. If the guide carriage 124 is formed along the exterior surface of the cannula 14 (as shown in FIGS. 7-8), then the cannula 14 should preferably be positioned so that the guide carriage 124 does not extend in a trans-valvular fashion. For example, with reference to FIG. 6, the guide carriage 124 should be positioned wholly within the left ventricle such that the pulsatile blood flow during beating heart procedures will not inadvertently pass through the side lumen 126 and pass through the aortic valve.

The intravascular blood pump system 120 is constructed in virtually the same manner as the intravascular blood pump system 10 shown and described above, with the exception of the location of the respective guide mechanisms 16, 122. More specifically, because the guide mechanism 122 is disposed along the side of the cannula 14, there is no need to form a central lumen extending through the blood pump 12, drive cable assembly 18, purge fluid delivery system 26, and motor coupler 24 as detailed above with regard to the intravascular blood pump system 10. As such, these components need not be specially machined or molded to include such central lumens as was required with the intravascular blood pump system 10 set forth above.

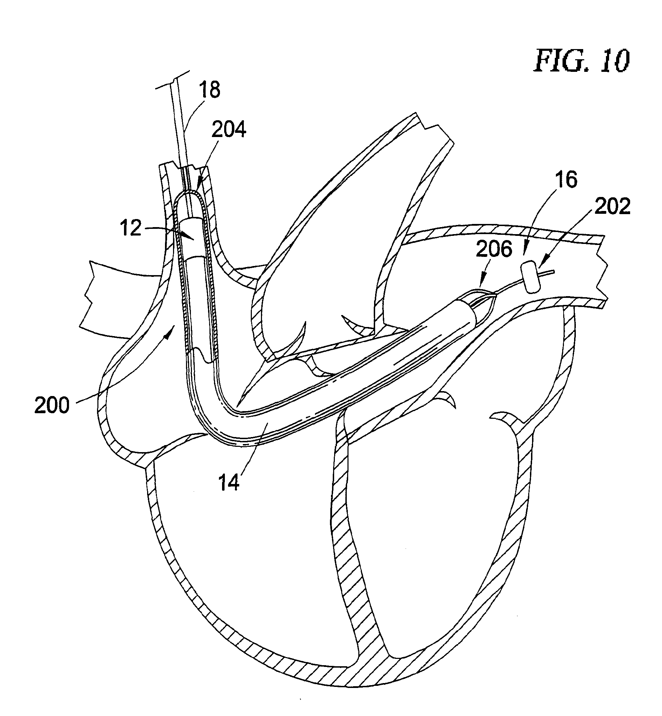

While this invention has been shown in use largely in during left-heart applications, it is to be readily appreciated that this does not limit the applications of this invention for use in left heart support only. Rather, the guidable intravascular blood pump of the present invention can be utilized in right-heart support applications and a wide variety of other applications apparent to those skilled in the art. For example, with reference to FIG. 10, shown is an intravascular blood pump 200 (of the type shown and described above with reference to FIGS. 2-5) configured for use in a right-heart support application. In this embodiment, the intravascular blood pump system 200 is equipped, by way of example, with an "over-the-wire" guide mechanism 16 comprising a balloon catheter 202. It is to be readily appreciated that, although shown and described below in terms of an embodiment of the type shown in FIGS. 2-5, the intravascular blood pump systems 120, 130 disclosed herein may also be configured for use in right-heart applications. Such right-heart configurations, and others apparent to those skilled in the art based on the broad principles enumerated in this application, are contemplated as being within the scope of the present invention.

The intravascular blood pump system 200 is shown positioned within the heart, such as may be advantageous to provide right heart support during beating heart surgery. To position the guidable intravascular blood pump system 200 in the right heart according to the present invention, a suitable guide element (such as balloon catheter 202) is first advanced to a desired location within the heart via the "sail" action of an inflated balloon. After the balloon catheter 202 is located in the desired position (such as in the pulmonary artery as shown), the intravascular blood pump system 200 according to the present invention may be advanced over the balloon catheter 202 and guided into a desired arrangement. For right heart support, this would involve advancing the pump 12 and cannula 14 over the balloon catheter 202 until the fluid inlet 204 is disposed within the vena cava (or right atrium) and the fluid outlet 206 is positioned within the pulmonary artery. The pump 12 may then be selectively (i.e. automatically or on-demand) controlled to transport blood from the vena cava (or right atrium) in a trans-valvular fashion through the tricuspid valve, the right ventricle, and the pulmonary valve for deposit within the pulmonary artery. Providing right-heart support during beating heart surgery advantageously overcomes conditions where cardiac output may become compromised during beating heart surgery, such as when the heart is lifted to gain access to posterior vessels, thereby avoiding the need for cardiopulmonary bypass.

It is also contemplated as part of the present invention that the guidable intravascular blood pump systems can be introduced into the patient's vasculature to achieve the intravascular access into the right or left heart through any number of access points, including but not limited to the internal jugular vein, the brachiocephalic vein, carotid artery, axillary artery, femoral vein, femoral artery, and subclavian artery. The intravascular blood pump systems of the present invention may also be introduced via direct introduction, such as into the aorta, the atria, and the ventricles. As is well known in the art, such intravascular access may be achieved percutaneously through the use of the Seldinger technique or directly through the use of minimally invasive access techniques.

Those skilled in the art will also appreciate that, although shown and described above in terms of "axial flow," the present invention is not limited to the axial flow type intravascular blood pumps. Rather, the intravascular blood pumps 12 may comprise any number of suitable types of intravascular blood pumps, including but not limited to so-called "mixed flow" intravascular blood pumps, without departing from the scope of the present invention.

With regard to the arrangements shown in FIGS. 10-17, it is furthermore contemplated that the guide catheter 132 may be separable from the conduit assembly 134 after the pump assembly 136 is docked within the shroud 36 to form the pump 12 at the desired location within the circulatory system of the patient. This may be accomplished by providing the guide catheter 132 in a detachable fashion via any number of suitable arrangements. By removing the guide catheter 132 after the pump 12 assembled, wound management of the access point into the patient's vasculature may be improved. This is due, in part, to the substantial reduction in size of the device extending into the patient (i.e. the drive cable assembly 18 as opposed to the larger diameter guide catheter 132).

It is also contemplated to incorporate various pressure sensing and/or guidability features into at least one of the cannula 14 and pump 12. Such features may include, but are not necessarily limited to, those shown and described in commonly-owned and co-pending U.S. Patent Application No. 09/280,988 (filed March 30, 1999) entitled "Steerable Cannula," and U.S. Patent Application No. 09/280,970 (filed March 30, 1999) entitled "Pressure Sensing Cannula." The pressure sensing in the system of the invention includes the use of a fluid-filled lumen. The guidability features may include, but are not necessarily limited to, the use of side lumens and deformable materials (i.e. Nitonol).

Various pump and cannula arrangements have been described and shown above for providing right and/or left heart support wherein blood is deliberately re-routed through and past the right and/or left ventricle in an effort to reduce the volume of blood to be pumped by the particular ventricle. While "unloading" the ventricles in this fashion is preferred in certain instances, it is to be readily understood that the pump and cannula arrangements described herein may also be employed to "preload" the ventricles. Ventricular preloading may be accomplished by positioning the outflow cannula from the pump into a given ventricle such that the pump may be employed to fill or preload the ventricle with blood. This may be particularly useful with the right ventricle. On occasion, the right ventricle is not supplied with sufficient levels of blood from the right atrium such that, upon contraction, the right ventricle delivers an insufficient quantity of blood to the pulmonary artery. This may result when the right ventricle and/or right atrium are in a stressed or distorted condition during surgery. Preloading overcomes this problem by actively supplying blood into the right ventricle, thereby facilitating the delivery of blood into the pulmonary artery. The same technique can be used to preload the left ventricle and thus facilitate the delivery of blood from the left ventricle into the aorta.

## Claims

1. An intravascular blood pump system for ventricular assist comprising:
an intravascular blood pump (12) having a cannula (14) coupled distally thereto, the intravascular blood pump (12) including a pump body (34), a rotor shroud (36) having flow ports (38) and an internally disposed rotor (44) having a shaft rotatably disposed within the pump body (34) and an impeller rotatably disposed within the rotor shroud (36);
wherein the cannula (14) comprises a plurality of ports (40) about its distal region, and an end port (42);
a blood pressure sensing feature incorporated into at least one of the intravascular blood pump (12) and the cannula (14), wherein said blood pressure sensing feature comprises at least one of a fluid filled lumen, a piezo-electric pressure sensing element, a strain gauge, and analysis of the torque/current relationship based on the dynamic pressure differential between the inlet and outlet of the blood pump (12);
a guide wire (22) capable of being introduced into the vascular system of a patient; and
a guide mechanism (16), adapted to guide said intravascular blood pump and cannula to a predetermined location within the circulatory system of a patient, which is
(a) an "over-the-wire" guide mechanism wherein a central lumen is formed through at least a portion of the intravascular blood pump (12) such that the guide wire (22) may be progressed therethrough; or
(b) a "rapid exchange" guide mechanism wherein a side lumen is formed along a length of at least one of the intravascular blood pump (12) and the cannula (14) and is dimensioned to slideably receive the guide wire (22), such that the blood pump and cannula may be advanced along the guide wire (22).

2. The intravascular blood pump system of Claim 1 and further, wherein said cannula (14) is coupled to said shroud (36) of said intravascular blood pump (12)

3. The intravascular blood pump system of Claim 1 and further, wherein said intravascular blood pump (12) and cannula (14) may be selectively advanced to said predetermined location within the vasculature of the patient by first passing said guide wire (22) to said predetermined location and thereafter sliding said intravascular blood pump (12) and cannula (14) along said guide wire (22) to said predetermined location.

4. The intravascular blood pump system of Claim 1, wherein the cannula (14) is reinforced with a spiral wound wire embedded in a wall of the cannula (14).

5. The intravascular blood pump system of Claim 1, wherein the cannnula (14) is configured to be placed in a trans-valvular position for left-heart ventricular assist procedures.

6. The Intravascular blood pump system of Claim 1, wherein said cannula (14) comprises a diameter from 3 millimeters (9 French) to 10 millimeters (30 French).

7. The intravascular blood pump system of Claim 1, wherein the lumen of the guide mechanism is formed within a wall of the cannula (14).

8. The intravascular blood pump of claim 1, wherein the guide mechanism (16) is the "over-the-wire" guide mechanism.

9. The intravascular blood pump of claim 8, wherein the central lumen extends through the blood pump (12), cannula (14), and a drive cable (18).

10. The intravascular blood pump system of Claim 1, wherein the guide mechanism (16) is a rapid exchange guide mechanism (122).

11. The intravascular blood pump system of claim 10, wherein the guide mechanism includes a guide carriage(124) formed along at least a portion of the cannula (14) and through which the lumen extends.

## Patentansprüche

1. Intravaskuläres Blutpumpsystem zur ventrikulären Unterstützung, umfassend:
eine intravaskuläre Blutpumpe (12) mit einer distal an diese gekoppelten Kanüle (14), wobei die intravaskuläre Blutpumpe (12) ein Pumpengehäuse (34), eine Rotorumhüllung (36) mit Fließöffnungen (38) und einen im Inneren angeordneten Rotor (44) enthält, der eine Welle hat, die drehbar innerhalb des Pumpengehäuses (34) angeordnet ist, sowie ein Flügelrad, das drehbar innerhalb der Rotorumhüllung (36) angeordnet ist,
wobei die Kanüle (14) eine Vielzahl von Öffnungen (40) um ihren distalen Bereich herum und eine Endöffnung (42) umfasst,
ein Blutdruckmessmerkmal, das in mindestens einem Element ausgewählt aus der intravaskulären Blutpumpe (12) und der Kanüle (14) aufgenommen ist, wobei das genannte Blutdruckmessmerkmal mindestens ein Element ausgewählt aus einem fluidgefüllten Lumen, einem piezoelektrischen Druckmesselement, einem Dehnungsmessstreifen und einer Analyse des Drehmoment/Strom-Verhältnisses basierend auf dem dynamischen Druckdifferential zwischen dem Einlass und dem Auslass der Blutpumpe (12) umfasst,
einen Führungsdraht (22), der dazu geeignet ist, in das vaskuläre System eines Patienten eingeführt zu werden, und
einen Führungsmechanismus (16), der dazu geeignet ist, die genannte intravaskuläre Blutpumpe und Kanüle zu einer vorgegebenen Stelle innerhalb des Blutkreislaufsystems eines Patienten zu führen, wobei der Mechanismus
(a) ein "Over-the-Wire"-Führungsmechanismus ist, wobei ein zentrales Lumen durch mindestens einen Abschnitt der intravaskulären Blutpumpe (12) hindurch ausgebildet ist, derart, dass der Führungsdraht (22) durch dieses fortbewegt werden kann, oder
(b) ein "Schnellwechsel"-Führungsmechanismus, wobei ein Seitenlumen entlang einer Länge mindestens eines Elements ausgewählt aus der intravaskulären Blutpumpe (12) und der Kanüle (14) ausgebildet und so bemessen ist, dass es den Führungsdraht (22) verschiebbar aufnimmt, derart, dass die Blutpumpe und die Kanüle entlang des Führungsdrahtes (22) vorgeschoben werden können.

2. Intravaskuläres Blutpumpsystem nach Anspruch 1, und wobei ferner die genannte Kanüle (14) mit der genannten Umhüllung (36) der genannten intravaskulären Blutpumpe (12) gekoppelt ist.

3. Intravaskuläres Blutpumpsystem nach Anspruch 1, und wobei ferner die genannte intravaskuläre Blutpumpe (12) und die Kanüle (14) selektiv zu der genannten vorgegebenen Stelle innerhalb des Gefäßsystems des Patienten vorgeschoben werden können, indem zunächst der genannte Führungsdraht (22) zu der genannten vorgegebenen Stelle geführt wird und daraufhin die genannte intravaskuläre Blutpumpe (12) und die Kanüle (14) entlang dem genannten Führungsdraht (22) zu der genannten vorgegebenen Stelle geschoben werden.

4. Intravaskuläres Blutpumpsystem nach Anspruch 1, wobei die Kanüle (14) mit einem spiralgewickelten Draht verstärkt ist, der in einer Wand der Kanüle (14) eingebettet ist.

5. Intravaskuläres Blutpumpsystem nach Anspruch 1, wobei die Kanüle (14) derart konfiguriert ist, dass sie in einer transvalvulären Position für linksventrikuläre Unterstützungsverfahren platziert werden kann.

6. Intravaskuläres Blutpumpsystem nach Anspruch 1, wobei die genannte Kanüle (14) einen Durchmesser von 3 Millimeter (9 Charriäre) bis 10 Millimeter (30 Charriäre) umfasst.

7. Intravaskuläres Blutpumpsystem nach Anspruch 1, wobei das Lumen des Führungsmechanismus innerhalb einer Wand der Kanüle (14) ausgebildet ist.

8. Intravaskuläres Blutpumpsystem nach Anspruch 1, wobei der Führungsmechanismus (16) der sogenannte "Over-the-Wire"-Führungsmechanismus ist.

9. Intravaskuläres Blutpumpsystem nach Anspruch 8, wobei sich das zentrale Lumen durch die Blutpumpe (12), die Kanüle (14) und ein Antriebskabel (18) erstreckt.

10. Intravaskuläres Blutpumpsystem nach Anspruch 1, wobei der Führungsmechanismus (16) der Schnellwechsel-Führungsmechanismus (122) ist.

11. lntravaskuläres Blutpumpsystem nach Anspruch 10, wobei der Führungsmechanismus einen Führungsschlitten (124) enthält, der entlang wenigstens eines Abschnitts der Kanüle (14) ausgebildet ist und durch den sich das Lumen erstreckt.

## Revendications

1. Système de pompe à sang intra-vasculaire pour assistance ventriculaire, comprenant
- une pompe à sang intra-vasculaire (12) munie d'une canule (14) couplée à celle-ci de manière distale, la pompe à sang intra-vasculaire (12) comportant un corps de pompe (34), une enveloppe de rotor (36) présentant ports d'écoulement (38) et un rotor (44) disposé à l'intérieur possédant un arbre disposé pour tourner à l'intérieur du corps de pompe (34) et une hélice disposée pour tourner à l'intérieur de l'enveloppe de rotor (36) ;
dans lequel la canule (14) comprend une pluralité de ports (40) au niveau de sa région distale, et un port d'extrémité (42) ;
- un instrument de mesure de la tension artérielle intégré dans au moins l'une de la pompe à sang intra-vasculaire (12) et de la canule (14), dans lequel ledit instrument de mesure de la tension artérielle comprend au moins l'un parmi une lumière remplie de fluide, un élément de détection de pression piézo-électrique, une jauge de contraintes et une analyse de la relation couple/courant en se fondant sur le différentiel de pression dynamique entre l'entrée et la sortie de la pompe à sang (12),
- un fil guidage (22) apte à être introduit dans le système vasculaire d'un patient ; et
- un mécanisme de guidage (16) adapté à guider ladite pompe à sang intra-vasculaire et ladite canule jusqu'à un emplacement prédéterminé, à l'intérieur du système circulatoire d'un patient, qui est
(a) un mécanisme de guidage « à passage de guide-fil » dans lequel une lumière centrale est formée à travers au moins une portion de la pompe à sang intra-vasculaire (12), de telle sorte que le fil de guidage (22) puisse progresser à travers celle-ci ; ou
(b) un mécanisme de guidage « à échange rapide » dans lequel une lumière latérale est formée sur une longueur d'au moins l'une de la pompe à sang intra-vasculaire (12) et de la canule (14) et est dimensionnée de manière à recevoir, par glissement, le fil de guidage (22), de telle sorte que la pompe à sang et la canule puissent être avancées le long du fil de guidage (22).

2. Système de pompe à sang intra-vasculaire selon la revendication 1 et, en outre, dans lequel ladite canule (14) est couplée à ladite enveloppe (36) de ladite pompe à sang intra-vasculaire (12).

3. Système de pompe à sang intra-vasculaire selon la revendication 1 et, en outre, dans lequel ladite pompe à sang intra-vasculaire (12) et ladite canule (14) peuvent être avancées, de manière sélective, jusqu'au dit emplacement prédéterminé à l'intérieur des vaisseaux du patient en faisant tout d'abord passer ledit fil de guidage (22) jusqu'au dit emplacement prédéterminé et ensuite en faisant glisser ladite pompe à sang intra-vasculaire (12) et ladite canule (14) le long dudit fil de guidage (22) jusqu'au dit emplacement prédéterminé.

4. Système de pompe à sang intra-vasculaire selon la revendication 1, dans lequel la canule (14) est renforcée par un fil enroulé en spirale intégré dans une paroi de la canule (14).

5. Système de pompe à sang intra-vasculaire selon la revendication 1, dans lequel la canule (14) est configurée de manière à être placée dans une position trans-valvulaire pour des procédures d'assistance sur le ventricule gauche.

6. Système de pompe à sang intra-vasculaire selon la revendication 1, dans lequel ladite canule (14) présente un diamètre compris entre 3 millimètres (9 sur la jauge française) et 10 millimètres (30 sur la jauge française).

7. Système de pompe à sang intra-vasculaire selon la revendication 1, dans lequel la lumière du mécanisme de guidage est formée à l'intérieur d'une paroi de la canule (14).

8. Système de pompe à sang intra-vasculaire selon la revendication 1, dans lequel le mécanisme de guidage (16) est le mécanisme de guidage « à passage de guide-fil ».

9. Système de pompe à sang intra-vasculaire selon la revendication 8, dans lequel la lumière centrale s'étend à travers la pompe à sang (12), la canule (14) et un câble d'entraînement (18).

10. Système de pompe à sang intra-vasculaire selon la revendication 1, dans lequel le mécanisme de guidage (16) est le mécanisme de guidage à échange rapide (122).

11. Système de pompe à sang intra-vasculaire selon la revendication 10, dans lequel le mécanisme de guidage inclut un support de guidage (124) formé le long d'au moins une portion de la canule (14) et à travers lequel s'étend la lumière.
